# FASCICULE DE BREVET EUROPEEN

(11) **EP 1 067 966 B1**
(45) Date de publication et mention de la délivrance du brevet: **12.05.2004**
(21) Numéro de dépôt: 99909028.5
(22) Date de dépôt: 17.03.1999
(51) Int. Cl.: A61K 47/36, A61K 9/10, A61P 27/06

(54) **COMPOSITION OPHTALMIQUE COMPRENANT UN BETA-BLOQUANT**
BETABLOCKER ENTHALTENDE OPHTALMISCHE ZUSAMMENSETZUNGEN
OPHTHALMIC COMPOSITION COMPRISING A BETA-BLOCKER

(30) Priorité: 09.04.1998 FR 9804489
(43) Date de publication de la demande: 17.01.2001
(73) Titulaire: Laboratoire Chauvin S.A., 34009 Montpellier Cédex 1 (FR)
(72) Inventeur: MAURIN, Florence, F-34570 Vailhauques (FR); LATOUR, Elisabeth, F-34070 Montpellier (FR); COQUELET, Claude, F-34470 Pérols (FR)
(74) Mandataire: Bernasconi, Jean Raymond
(86) Numéro de dépôt international: PCT/FR1999/000612
(87) Numéro de publication internationale: WO 1999/052559

(56) Documents cités:
- WO-A-95/05803
- WO-A-95/35073
- WO-A-98/11874
- US-A- 5 318 780
- GHOSH L. ET AL: "Development and evaluation of an oral controlled release multiple emulsion drug delivery system of a specific beta blocker, metoprolol tartrate" BOLL. CHIM. FARM., vol. 135, no. 8, 1996, pages 468-471, XP002089212

## Description

La présente invention concerne des compositions ophtalmiques comprenant un β-bloquant.

Des compositions ophtalmiques comprenant des β-bloquants sont utilisées dans le traitement des hypertensions oculaires et du glaucome. Comme exemples, on peut citer des collyres à base de cartéolol, timolol, béfunolol, métipranolol, lévobunolol, pindolol ou bétaxolol. Ces solutions ont une durée d'action relativement courte, de sorte que l'administration de ces collyres doit être renouvelée au cours d'une journée.

Cohen et al. (Journal of Control Release, 44, 201, 1997) ont par ailleurs proposé des solutions aqueuses de pilocarpine contenant un alginate de sodium à teneur élevée en acide guluronique, qui forment un gel lorsque la solution est appliquée sur l'oeil (apparemment en raison de l'action des ions calcium présents dans le liquide lacrymal) et permettent d'obtenir une durée d'action prolongée.

Les inventeurs ont cependant constaté que si l'on applique ce système aux β-bloquants, c'est-à-dire si l'on ajoute un β-bloquant à une solution d'alginate de sodium, on n'obtient pas d'effet prolongé après administration de la solution.

Les inventeurs ont en revanche découvert que si l'on ajoute un β-bloquant à une composition aqueuse contenant de l'acide alginique et que l'on remonte le pH par addition d'une base alcaline, on obtient une solution qui permet d'obtenir un effet prolongé après administration et qui de ce fait permet une administration journalière unique.

La présente invention a ainsi pour objet une composition ophtalmique comprenant en solution un β-bloquant, cette composition étant obtenue par dissolution dans l'eau du β-bloquant en présence d'acide alginique et addition d'une base alcaline, pour obtenir une solution ayant un pH de 6 à 8.

La présente invention a également pour objet un procédé de préparation d'une composition ophtalmique, consistant à dissoudre dans de l'eau un β-bloquant en présence d'acide alginique et à ajouter une base alcaline pour amener son pH à une valeur de 6 à 8.

L'acide alginique est de préférence un acide alginique ayant une teneur en acide guluronique d'au moins 40 % et, de préférence, d'au moins 65 %, par exemple un acide alginique ayant une teneur en acide guluronique de 65 à 75 %.

Les β-bloquants présents dans la composition peuvent être choisis notamment parmi le cartéolol, le timolol, le béfunolol, le métipranolol, le lévobunolol, le pindolol ou le bétaxolol.

Les β-bloquants sont ajoutés en général sous forme de sels avec des acides pharmaceutiquement acceptables.

Ils sont présents généralement à des concentrations de 0,01 à 5 % et de préférence de 0,1 à 2 % (exprimées sous forme de base) du poids de la composition finale.

L'acide alginique est utilisé à des concentrations de 0,01 à 5 % et, de préférence, entre 0,1 et 2 % du poids de la composition finale.

Le rapport pondéral acide alginique/β-bloquant (exprimé sous forme de base) est en général de 0,1 à 20 et de préférence de 0,2 à 10.

La base alcaline est un hydroxyde de métal alcalin ou un sel basique de métal alcalin et est de préférence de l'hydroxyde de sodium.

La composition peut en outre contenir un mélange tampon tel qu'un tampon phosphate, un agent d'isotonicité tel que du chlorure de sodium, des stabilisants tels qu'un antioxydant et/ou un agent chélatant (par exemple l'EDTA), ainsi qu'un conservateur tel que du chlorure de benzalkonium.

On donnera ci-après des résultats d'essais mettant en évidence les effets obtenus avec les compositions selon l'invention.

### 1) Essais comparatifs avec du cartéolol

Les modes opératoires utilisés ont été les suivants :

### Mode opératoire 1

On met en suspension de l'acide alginique dans environ 50 ml d'eau purifiée et on y dissout du chlorhydrate de cartéolol (2g). Le pH est alors voisin de 3.

On ajoute le tampon phosphate (0,1 g d'hydrogénophosphate de sodium dodécahydraté et 0,04 g de dihydrogénophosphate de sodium dihydraté) puis du chlorure de sodium. Le pH atteint alors une valeur voisine de 3,5.

On ajuste le pH à 6,8 avec une solution d'hydroxyde de sodium 1N et on incorpore du chlorure de benzalkonium (0,005 g).

On ajuste enfin le volume à 100 ml par addition d'eau purifiée.

### Mode opératoire 2

On dissout de l'alginate de sodium dans environ 50 ml d'eau purifiée, la valeur du pH est voisine de 8.

Dans 40 ml d'eau purifiée, on dissout du chlorhydrate de cartéolol (2 g), du tampon phosphate et du chlorure de sodium (le pH de la solution est voisin de 6,8).

On ajoute cette solution à la solution aqueuse d'alginate de sodium et ajuste le pH à 6,8 par addition d'acide chlorhydrique dilué.

On incorpore du chlorure de benzalkonium (0,005 g) et ajuste le volume final à 100 ml par addition d'eau purifiée.

### Mode opératoire 3

On met en suspension de l'acide alginique dans environ 50 ml d'eau purifiée puis ajuste le pH à 8 par addition d'une solution d'hydroxyde de sodium 1N.

Dans 40 ml d'eau purifiée, on dissout du chlorhydrate de cartéolol (2 g), du tampon phosphate et du chlorure de sodium (le pH de la solution est voisin de 6,8).

On ajoute cette dernière solution à la solution d'acide alginique neutralisée et on ajuste le pH à 6,8 par addition d'acide chlorhydrique dilué.

On incorpore du chlorure de benzalkonium (0,005 g) et on ajuste le volume final à 100 ml par addition d'eau purifiée.

Ces compositions ont été testées dans un modèle d'hypertension oculaire provoquée chez le lapin.

### Animaux

Les expériences ont été réalisées sur des lapins mâles albinos Néo-Zélandais provenant de l'Elevage Charles River France (St Aubin les Elbeuf, 76140 Cléon) acclimatés pendant cinq jours minimum dans l'animalerie (température : 19 ± 2° C ; humidité relative : 55 ± 10 %; éclairage : 12 heures de jour - 12 heures d'obscurité).

### Hypertension oculaire provoquée par surcharge aqueuse

Les animaux ont été privés de nourriture la veille des expériences. Le jour de l'expérience, les animaux ont été conditionnés à la stabulation en boîte à contention et à la mesure de tension intraoculaire (TIO) jusqu'à l'obtention d'une valeur de base stable. La TIO a été mesurée à l'aide d'un pneumatonographe à aplanation ALCON ou d'un pneumatonomètre MENTOR® (modèle 30 Classic), sans anesthésie locale préalable.

Les préparations ophtalmiques testées ont ensuite été administrées dans un oeil, sous un volume de 25 µl, à différents temps avant la surcharge aqueuse : l'oeil controlatéral n'a reçu aucun traitement et a servi de contrôle.

L'hypertension oculaire expérimentale a été induite dans les deux yeux par administration orale d'eau à 37° C (70 ml/kg en moins de 30 secondes ; 200 ml pour les lapins d'un poids supérieur à 3 kg). La TIO a été mesurée dans les deux yeux avant, puis toutes les dix minutes pendant une heure après la surcharge hydrique.

L'activité hypotensive oculaire des préparations testées est calculée à partir des augmentations de TIO dans l'oeil traité et l'oeil contrôle, au pic de l'hypertension expérimentale ; elle est exprimée en pourcentage d'inhibition de l'hypertension (moyenne ± écart-type).

### Estimation de l'activité maximale et de la durée d'action des préparations testées

L'hypertension oculaire provoquée par surcharge aqueuse est une hypertension aiguë puisque la TIO retourne à sa valeur de base en moyenne une heure après la surcharge aqueuse. Le pic d'activité hypotensive et la durée d'action des préparations testées ne peuvent donc être évalués en une seule expérience ; une estimation assez précise de ces deux paramètres est obtenue de manière indirecte sur plusieurs expériences en faisant varier le moment d'administration des préparations par rapport à la surcharge hydrique (temps de prétraitement).

Les résultats sont rassemblés dans le tableau ci-après.

**TABLEAU**

| **Activité hypotensive oculaire exprimée en % d'inhibition de l'hypertension induite par surcharge hydrique chez le lapin** | | | | |
|---|---|---|---|---|
| Composition | Dérivé alginique | Temps de prétraitement | | |
| | | 1 h | 6 h | 8h |
| 1 | - | 25,5±6,2 (13)** | 15,2±8,2 (8) | 0,6±5,6 (4) |
| 2 (1)* | acide alginique 1% (G= entre 65 et 75 %) | 24,4±4,8 (5) | 13,2±6,0 (5) | 10,0±6,1 (4) |
| 3 (1) | acide alginique 1 % ( G = 39 %) | | 12,5±2,1 (4) | 3,6±4,0 (5) |
| 4 (2) | alginate de sodium1% LVG (G = entre 65 et 75%) | | | 1,9±1,5 (4) |
| 5 (2) | alginate de sodium 1% MVG (G = entre 65 et 75 %) | | | -0,2±1,6 (4) |
| 6 (3) | alginate de sodium formé in situ à partir d'acide alginique 1 % (G = entre 65 et 75 %) | | | 1,6±3,5 (5) |

| | | | | |
|---|---|---|---|---|
| *( ) mode opératoire | | | | |
| **( ) nombre d'animaux | | | | |
| G : teneur en acide guluronique | | | | |
| LVG : faible viscosité, MVG = viscosité moyenne. | | | | |

Une solution ophtalmique à base de chlorhydrate de cartéolol 2 % sans dérivé alginique (composition 1) n'a plus d'activité après 8 h00 de prétraitement.

Une composition ophtalmique correspondante selon l'invention (composition 2) contenant 1 % d'acide alginique (acide alginique contenant entre 65 et 75 % d'acide guluronique) présente encore au temps 8h00 une inhibition significative de l'hypertension oculaire.

L'utilisation d'acide alginique à la concentration de 1% dont la teneur en acide guluronique est de 39% conduit à une composition en chlorhydrate de cartéolol à 2% (composition 3) induisant une réduction de l'hypertension oculaire plus faible au temps 8h00 qu'une composition contenant 1% d'acide alginique dont la teneur en acide guluronique est élevée, entre 65 et 75%.

L'utilisation d'alginate de sodium à la concentration de 1% dont la teneur en acide guluronique est comprise entre 65 et 75% ne permet pas d'obtenir des compositions ophtalmiques à base de chlorhydrate de cartéolol 2% présentant une inhibition de l'hypertension oculaire après 8h00 de prétraitement. Ces résultats sont observés avec des alginates de sodium de faible viscosité et de viscosité moyenne (compositions 4 et 5).

Si, lors de la préparation de la composition ophtalmique, l'acide alginique (à la concentration de 1%) est salifié par addition d'hydroxyde de sodium jusqu'à pH 8 avant l'addition du chlorhydrate de cartéolol, aucune inhibition de l'hypertension oculaire induite n'est observée après 8h00 de prétraitement (composition 6). La formation d'alginate in situ à partir d'acide alginique de teneur en acide guluronique comprise entre 65 et 75% ne permet pas de retrouver l'effet pharmacologique observé avec la composition 2.

### 2) Essais avec des β-bloquants autres que le cartéolol

### a) Timolol

Une étude de l'activité hypotensive oculaire en fonction de la concentration en acide alginique (% exprimé en poids par rapport au poids de la composition finale) a été menée sur le timolol à la concentration de 0,5% (exprimée en base ; soit 0,683% de maléate de timolol) dans des préparations selon le mode opératoire 1 en utilisant un acide alginique à teneur en acide guluronique élevée (G supérieur à 65%) :

| **Activité hypotensive oculaire exprimée en % d'inhibition de l'hypertension induite par surcharge hydrique chez le lapin** | | |
|---|---|---|
| Acide alginique (en % du poids de la composition finale) | Temps de prétraitement | |
| | 6 h | 8h |
| 0* | 13,2 ± 4,3 (4) | 1,5 ± 4,4 (6) |
| 0,16 | | 8,5 ± 2,7 (4) |
| 0,32 | | 7,6 ± 4,2 (4) |
| 0,64 | | 8,2 ± 4,0 (5) |

| | | |
|---|---|---|
| * collyre commercialisé contenant 0,5% de timolol exprimé en base. | | |

Le collyre de référence commercialisé n'a plus d'activité après 8h00 de prétraitement. Par contre, l'effet est prolongé lorsque la composition contient de l'acide alginique.

### b) Pindolol

Une étude similaire a été réalisée avec le pindolol, utilisé sous forme base, à la concentration de 1%.

| Acide alginique (en % du poids de la composition finale) | Temps de prétraitement | |
|---|---|---|
| | 1 h | 4h |
| 0* | 13,0 ± 6,6 (4) | - 3,0 ± 8,7 (7) |
| 0,8 | | 10,3 ± 4,4 (4) |
| 1,6 | | 6,8 ± 1,7 (4) |

| | | |
|---|---|---|
| * collyre commercialisé contenant 1% de pindolol. | | |

Le collyre commercialisé n'a plus d'activité après 4h00 de prétraitement.

L'addition d'acide alginique permet d'augmenter la durée d'action du pindolol 1%.

### 3) Essais complémentaires sur le cartéolol

L'activité hypotensive oculaire du cartéolol en fonction de la concentration en acide alginique a été étudiée pour différentes concentrations de chlorhydrate de cartéolol : 0,5%-1% et 2% (soit respectivement 0,44%-0,89% et 1,78% exprimé sous forme base).

Les résultats sont donnés dans les trois tableaux suivants, comparativement aux collyres commercialisés sans acide alginique et contenant 0,5%-1% et 2% de chlorhydrate de cartéolol.

| **Chlorhydrate de cartéolol 0,5%** | | |
|---|---|---|
| Acide alginique (en % du poids de la composition finale) | Temps de prétraitement | |
| | 4 h | 6h |
| 0 | 11,4 ± 4,3 (4) | 1,3 ± 1,7 (4) |
| 0,25 | | 8,8 ± 4,7 (5) |
| 1 | | 5,9 ± 3,1 (5) |

| **Chlorhydrate de cartéolol 1%** | | |
|---|---|---|
| Acide alginique (en % du poids de la composition finale) | Temps de prétraitement | |
| | 6 h | 8h |
| 0 | 10,6 ± 3,9 (4) | - 0,2 ± 2,8 (5) |
| 0,5 | | 6,8 ± 2,8 (4) |
| 1 | | 10,1 ± 3,8 (4) |

| **Chlorhydrate de cartéolol 2%** | | |
|---|---|---|
| Acide alginique (en % du poids de la composition finale) | Temps de prétraitement | |
| | 6 h | 8h |
| 0 | 15,2 ± 8,2 (8) | 0,6 ± 5,6 (4) |
| 1 | | 8,0 ± 2,7 (4) |
| 2 | | 11,4 ± 2,2 (5) |

## Revendications

1. Composition ophtalmique comprenant en solution un β-bloquant, cette composition susceptible d'être obtenue par dissolution dans l'eau du β-bloquant en présence d'acide alginique et addition d'une base alcaline, pour obtenir une solution. ayant un pH de 6 à 8.

2. Composition selon la revendication 1, dans laquelle l'acide alginique a une teneur en acide guluronique d'au moins 40 %.

3. Composition selon la revendication 2, dans laquelle l'acide alginique a une teneur en acide guluronique d'au moins 65 %.

4. Composition selon l'une quelconque des revendications 1 à 3, dans laquelle le β-bloquant est choisi parmi le cartéolol, le timolol et le pindolol.

5. Composition selon l'une quelconque des revendications 1 à 4, dans laquelle le β-bloquant est présent à une concentration de 0,01 à 5 % en poids.

6. Composition selon l'une quelconque des revendications 1 à 5, dans laquelle l'acide alginique est présent à une concentration de 0,01 à 5 % en poids.

7. Composition selon l'une quelconque des revendications 1 à 6, dans laquelle le rapport en poids acide alginique/β-bloquant est de 0,1 à 20.

8. Composition selon la revendication 7, dans laquelle le rapport en poids acide alginique/β-bloquant est de 0,2 à 10.

9. Procédé de préparation d'une composition ophtalmique, consistant à dissoudre dans de l'eau un β-bloquant en présence d'acide alginique et à ajouter une base alcaline pour amener son pH à une valeur de 6 à 8.

## Patentansprüche

1. Ophtalmische Zubereitung, enthaltend einen β-Blocker in Lösung, wobei diese Zubereitung erhalten werden kann durch Lösen des β-Blockers in Gegenwart von Alginsäure und Zugabe einer Alkalibase, um eine Lösung mit einem pH-Wert von 6 bis 8 zu erhalten.

2. Zubereitung nach Anspruch 1, in der die Alginsäure einen Guluronsäuregehalt von mindestens 40 % aufweist.

3. Zubereitung nach Anspruch 2, in der die Alginsäure einen Guluronsäuregehalt von mindestens 65 % aufweist.

4. Zubereitung nach einem der Ansprüche 1 bis 3, in der der β-Blocker ausgewählt ist aus Carteolol, Timolol und Pindolol.

5. Zubereitung nach einem der Ansprüche 1 bis 4, in der der β-Blocker in einer Konzentration von 0,01 bis 5 Gew.% vorliegt.

6. Zubereitung nach einem der Ansprüche 1 bis 5, in der die Alginsäure in einer Konzentration von 0,01 bis 5 Gew.% vorliegt.

7. Zubereitung nach einem der Ansprüche 1 bis 6, in der das Gewichtsverhältnis Alginsäure/β-Blocker 0,1 bis 20 beträgt.

8. Zubereitung nach Anspruch 7, in der das Gewichtsverhältnis Alginsäure/β-Blocker 0,2 bis 10 beträgt.

9. Verfahren zur Herstellung einer ophtalmischen Zubereitung, bestehend aus Lösen eines β-Blockers in Gegenwart von Alginsäure und Zugeben einer Alkalibase, um ihren pH-Wert auf einen Wert von 6 bis 8 zu bringen.

## Claims

1. Ophthalmic composition comprising a β-blocker in solution, this composition capable of being obtained by dissolving the β-blocker in water in the presence of alginic acid and adding an alkaline base, in order to obtain a solution having a pH of 6 to 8.

2. Composition as claimed in Claim 1, in which the alginic acid has a guluronic acid content of at least 40%.

3. Composition as claimed in Claim 2, in which the alginic acid has a guluronic acid content of at least 65%.

4. Composition as claimed in any one of Claims 1 to 3, in which the β-blocker is chosen from among carteolol, timolol and pindolol.

5. Composition as claimed in any one of Claims 1 to 4, in which the β-blocker is present at a concentration of 0.01 to 5% by weight.

6. Composition as claimed in any one of Claims 1 to 5, in which the alginic acid is present at a concentration of 0.01 to 5% by weight.

7. Composition as claimed in any one of Claims 1 to 6, in which the alginic acid/ β-blocker weight ratio is from 0.1 to 20.

8. Composition as claimed in Claim 7, in which the alginic acid/ β-blocker weight ratio is from 0.2 to 10.

9. Method of preparing an ophthalmic composition, comprising dissolving a β-blocker in water in the presence of alginic acid solution and adding an alkaline base in order to bring its pH to a value of 6 to 8.
